# FASCICULE DE BREVET EUROPEEN

(11) **EP 0 429 376 B1**
(45) Date de publication et mention de la délivrance du brevet: **26.05.1993**
(21) Numéro de dépôt: 90420488.0
(22) Date de dépôt: 13.11.1990
(51) Int. Cl.: G01N 3/04, G01N 3/14

(54) **Dispositif pour mesurer les propriétés mécaniques d'un échantillon, notamment textile**
Vorrichtung zur Messung von mechanischen Eigenschaften einer Probe, bzw. Textilien
Apparatus for measuring the mechanical properties of a sample, especially textile

(30) Priorité: 17.11.1989 FR 8915379
(43) Date de publication de la demande: 29.05.1991
(73) Titulaire: TISSAGE ET ENDUCTION Serge FERRARI S.A., F-38110 La Tour Du Pin (FR)
(72) Inventeur: Allera, Rodolphe, F-38170 Seyssinet (FR); Ferrari, Sébastien, F-38910 Crobelin (FR)
(74) Mandataire: Laurent, Michel

(56) Documents cités:
- FR-A- 533 535
- GB-A- 2 029 971
- US-A- 2 568 731
- US-A- 2 850 895

## Description

L'invention concerne un dispositif apte à permettre la mesure des propriétés mécaniques d'un échantillon et notamment d'un échantillon textile.

Par "propriétés mécaniques", on entend principalement, mais non limitativement, le fluage et la ténacité en traction.

L'invention se rapporte plus particulièrement à un dispositif du type en question, apte à mesurer ces propriétés mécaniques selon une ou deux directions bien déterminées.

Jusqu'à présent, lorsque l'on désire procéder à la mesure du fluage d'un matériau, selon l'une de ses directions principales, on utilise un dynamomètre dont les deux mâchoires enserrent les deux extrémités d'une bande découpée dans ce matériau. La bande est découpée dans la direction selon laquelle on désire procéder à la mesure. De fait, si l'on désire procéder à la mesure du fluage selon deux directions bien définies, notamment perpendiculaires, par exemple selon la trame et selon la chaine s'il s'agit d'un matériau textile, on doit effectuer deux mesures consécutives ou utiliser un dynamomètre bi-axial. Ce type de dispositif est particulièrement coûteux car un tel appareil est en fait constitué de deux dynamomètres. Il s'avère de plus relativement long et difficile à mettre en oeuvre, donc il est impensable de pouvoir diffuser en masse un tel dispositif. Les documents US - A - 2 850 895 et GB - A - 2 029 971 décrivent des dispositifs permettant de mesurer les propriétés mécaniques de textiles dans lesquels les échantillons sont maintenus par des mâchoires. Dans le document US - A - 2 568 731, la traction sur les fils de l'échantillon textile est exercée, selon deux directions perpendiculaires, à l'aide de poids.

L'invention pallie ces inconvénients. Elle propose un dispositif apte à mesurer les propriétés mécaniques, notamment le fluage, d'un matériau se présentant sous forme de bande, peu coûteux à réaliser, facile à mettre en oeuvre et aussi fiable qu'un dynamomètre.

Ce dispositif, pour mesurer les propriétés mécaniques d'un échantillon sous forme de bande fixée à ses extrémités entre deux mâchoires,
- un socle sur lequel repose ladite bande et comprenant
- deux cylindres de renvoi parallèles, disposés de part et d'autre du socle et sur lequel est renvoyé ladite bande, sur les deux extrémités libres de laquelle s'exerce une traction de même intensité par l'intermédiaire des mâchoires ;
- deux biellettes pendulaires fixées respectivement d'une part aux mâchoires, et d'autre part à deux équerres par l'intermédiaire de deux chapes pendulaires, les deux équerres étant contenues dans le même plan, et solidaires de deux arbres parallèles, respectivement primaire et secondaire, et solidarisées entre elles au moyen d'un tirant dont les deux extrémités sont fixées aux équerres par l'intermédiaire de chapes, les deux équerres étant homothétiques l'une de l'autre dans le plan les contenant ;
- un bras de levier, solidaire de l'arbre primaire, dont la direction est toujours parallèle à la direction définie par la droite passant par respectivement l'axe de rotation de l'arbre primaire et de l'arbre secondaire et, l'axe de pivotement des chapes solidarisant les biellettes aux équerres ;
- un porte-poids, fixé à l'extrémité libre du bras de levier, et destiné à recevoir des masses de grandeur connues.

En d'autres termes, la présente invention se caractérise en ce que l'on associe entre eux des organes et éléments connus selon une architecture particulière, afin de générer au niveau des deux extrémités libres d'un échantillon sous forme de bande, une traction modulable de même intensité, ou un phénomène de fluage dont on peut alors mesurer les effets dans le temps.

Avantageusement en pratique :
- les arbres primaires et secondaires sont montés sur des paliers munis de roulements à bille ;
- la liaison des biellettes sur les mâchoires s'effectue au moyen de rotules ;
- les chapes sont fixées sur des roulements ;
- le socle recevant l'échantillon est chauffant ;
- la mesure des propriétés mécaniques, et notamment de fluage est effectuée au moyen d'un photomètre, ou tout système de traitement d'images, situé au-dessus du socle;
- les propriétés mécaniques sont mesurées selon deux directions perpendiculaires, la traction sur chacune des deux bandes perpendiculaires issues du matériau étant effectuée au moyen de deux dispositifs similaires à celui décrit précédemment et indépendant l'un de l'autre;
- les bras de levier sont verrouillés en position haute de manière indépendante au moyen d'un crochet muni d'une poignée agissant au niveau des arbres secondaires.

La manière dont l'invention peut être réalisée et les avantages qui en découlent, ressortiront mieux de l'exemple de réalisation donné à titre indicatif et non limitatif à l'appui des figures annexées.

La figure 1 est une vue schématique en perspective du dispositif conforme à l'invention, dans le cas de la mesure du fluage selon une seule direction.

La figure 2 est une vue schématique en perspective du dispositif dans le cas de la mesure du fluage selon deux directions perpendiculaires.

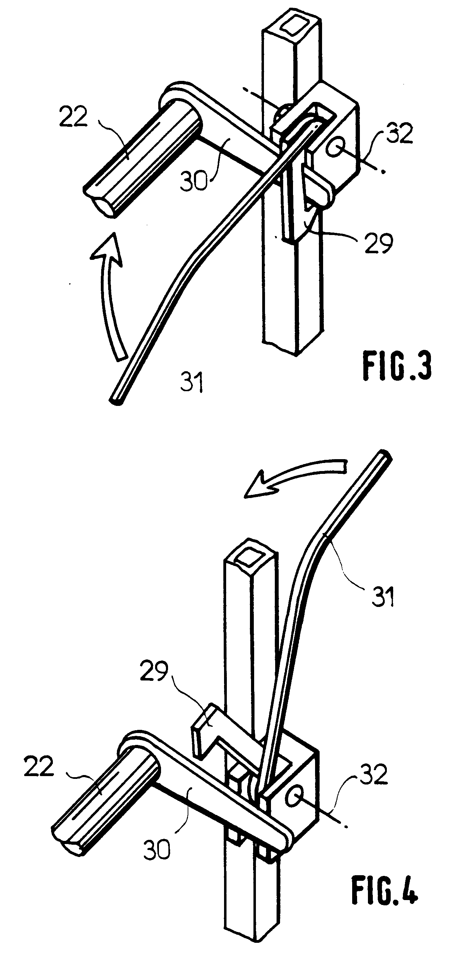

Les figures 3 et 4 représentent des vues schématiques partielles de l'organe de verrouillage du dispositif conforme à l'invention, respectivement en position opérationnelle et en position escamotée.

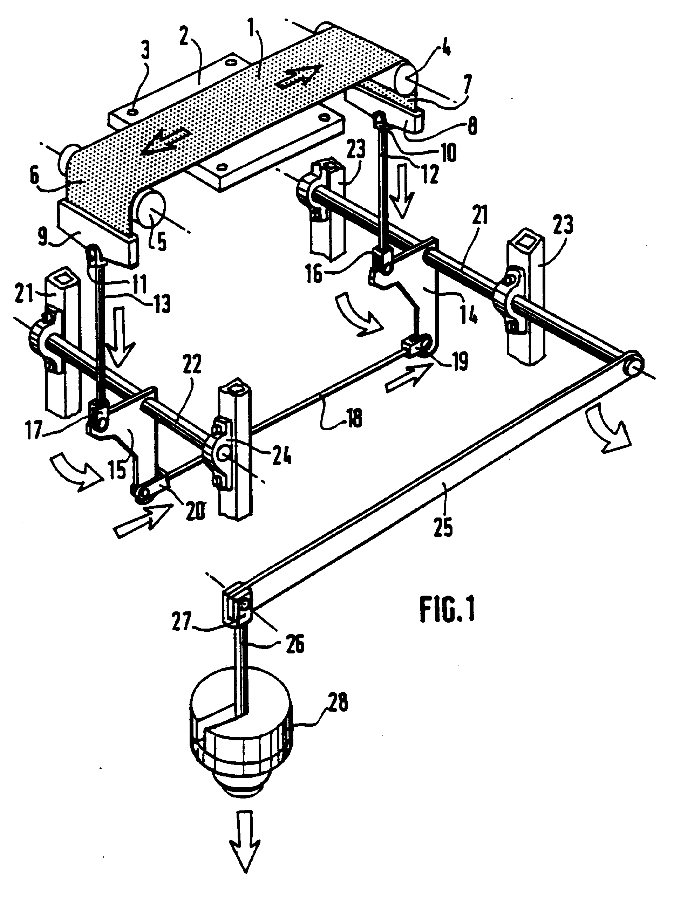

L'exemple décrit dans la figure 1 représente un dispositif apte à permettre la mesure du fluage d'un échantillon issu d'un matériau textile, par exemple d'une bache. Cette mesure s'effectue selon une seule direction, par exemple selon la chaine.

Une bande (1) de ce matériau est mise en place à plat sur le socle (2). Ce dernier est métallique et peut être avantageusement chauffant, notamment lorsque l'on désire effectuer des mesures de fluage en accéléré, permettant de la sorte une accélération du vieillissement du matériau, et donc d'observer sa tenue dans le temps.

Avantageusement mais non nécessairement, la bande (1) est maintenue en place sur le socle (2) au moyen d'une plaque transparente, par exemple réalisée en matière plastique, de même surface que le socle (2) et fixée sur ce dernier au moyen de vis dont on a représenté les orifices de fixation (3).

Les deux extrémités libres (6) et (7) de la bande (1) sont renvoyées sur des cylindres de renvoi (4) et (5) parallèles et libres en rotation. Ces deux extrémités libres (6) et (7) sont respectivement fixées à deux mâchoires (8) et (9) auto-bloquantes et auto-serrantes, couramment utilisées pour les sangles de sécurité dans le domaine de l'automobile et de l'aviation.

Chacune des deux mâchoires (8) et (9) est solidaire d'une équerre (14) et (15) et ce par l'intermédiaire d'une biellette (12) et (13). Ces biellettes sont avantageusement constituées d'un tendeur à vis, apte à assurer une légère pré-traction avant la mise en chargement, et à permettre une mise en place plus précise de la bande (1) sur le socle (2).

Ces tendeurs à vis (12) et (13) sont fixés aux mâchoires (8) et (9) au moyen de rotules (10) et (11) pendulaires. De la sorte, la traction exercée sur les deux extrémités (6) et (7) de la bande (1) d'un matériau dont on désire mesurer les propriétés mécaniques et notamment le fluage, est indépendante du positionnement de ladite bande dans les mâchoires. De plus, cette rotule permet le débattement pendulaire de la bielle qui dans le cas d'espèces est limitée à quelques degrés.

L'autre extrémité des tendeurs à vis (12) et (13) est fixée par l'intermédiaire d'une chape, respectivement (16) et (17) à une équerre (14) et (15). Cette chape est également pendulaire et est fixée sur les équerres (14) et (15) par l'intermédiaire d'une rotule, afin de limiter au maximum les frottements éventuels.

Les deux équerres (14) et (15) sont respectivement solidarisées à un arbre primaire (21) et secondaire (22), parallèles entre eux. Ces deux arbres (21) et (22) sont fixés au bâti portant l'ensemble du dispositif au moyen de paliers à roulements à billes (23) et (24).

Selon une caractéristique importante de l'invention, les deux équerres (14) et (15) sont situées dans un même plan, et homothétique l'une de l'autre dans ce plan. De fait, les deux équerres (14) et (15) sont identiques.

Selon une autre caractéristique de l'invention, les deux équerres (14) et (15) sont solidaires l'une de l'autre au moyen d'un tirant (18). Ce tirant est fixé aux deux équerres (14) et (15) au moyen de chapes (19) et (20) également montées sur rotules afin de limiter au maximum les forces de frottement. L'arbre primaire (21) est actionné au moyen d'un bras de levier (25) dont la direction est parallèle à la direction de la droite définie par le centre respectif des arbres primaire et secondaire (21) et (22) et l'axe de pivotement respectivement des rotules des chapes (16) et (17).

L'extrémité libre du bras de levier (25) comporte un porte-poids (26) monté pendulairement par l'intermédiaire d'une chape (17) et d'une rotule. Ce porte-poids est destiné à recevoir des masses (28) de grandeur connue.

Compte tenu de la structure de ce dispositif, lorsque l'on applique une masse sur le porte-poids (26), il en résulte compte tenu de la gravité, une rotation du bras de levier (25) dans le sens inverse des aiguilles d'une montre, tel que représenté dans la figure 1, provoquant une rotation de l'arbre primaire (21) dans le même sens. Dans l'exemple décrit, le rapport entre la force de traction sur les extrémités (6) et (7) de la bande (1) et l'intensité de la force créée par les masses (28) est égal à 5. Par exemple, une masse (28) de 60 kg génère une force de traction de 3000 Newtons. Il provoque d'une part la rotation de l'équerre (14) également dans le sens inverse des aiguilles d'une montre ayant pour effet, d'une part d'imprimer une traction sur l'extrémité (7) de la bande (1) et d'autre part d'induire la rotation de l'équerre (15), également en sens inverse des aiguilles d'une montre, cette dernière entrainant également une traction sur l'extrémité (6) de la bande (1).

Compte tenu des caractéristiques géométriques du montage, quelle que soit la position du bras de levier (25) sur sa course de rotation, et pour une masse (28) donnée, la traction exercée par les deux équerres (14) et (15) reste constante. En effet, par construction, la valeur de l'angle formé par les tendeurs à vis (12,13) par rapport à la verticale varie très peu, typiquement de l'ordre de quelques degrés. Or seul le cosinus de cet angle intervient dans le calcul de la contribution des masses (28), compte tenu que ce sont les moments des forces respectivement gravitationnelles (des masses (28)) et de traction sur les extrémités (6,7) qui maintiennent les système en équilibre. Typiquement, la variation des forces de traction sur lesdites extrémités (6,7) est de moins de 0,1% sur toute la course du bras de levier (25).

Le fluage est alors déterminé par mesure de l'écart séparant deux repères préalablement tracés au voisinage du centre de la bande sur le socle (2). Cet écart peut être mesuré manuellement au moyen de règles graduées mais avantageusement au moyen de photomètres non représentés, montés au-dessus du socle.

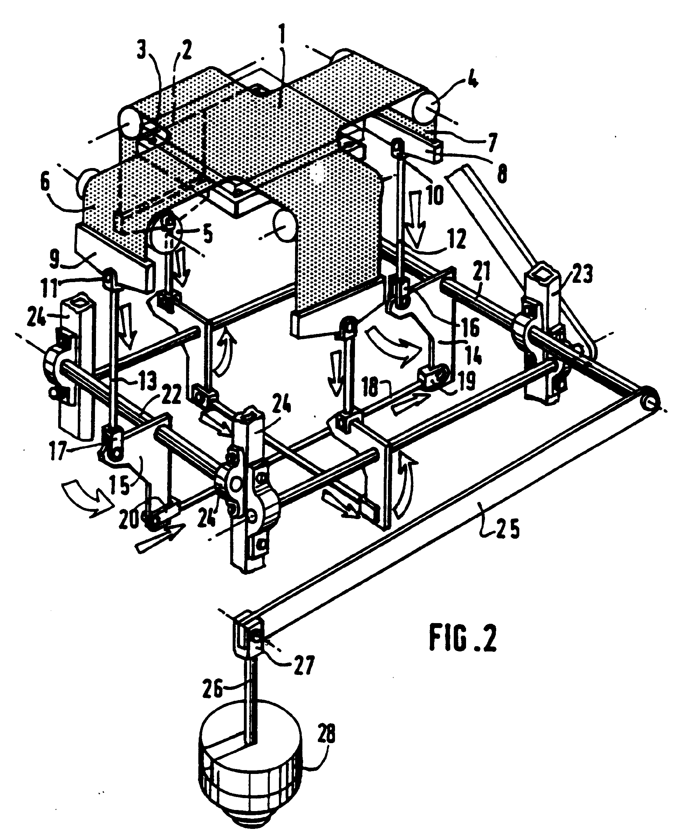

La présente invention peut également être appliquée pour mesurer les propriétés mécaniques d'un échantillon selon deux directions. Cette mesure peut être effectuée notamment selon deux directions perpendiculaires par exemple selon la trame et la chaine d'un tissu. Dans ce cas, comme on peut le voir dans la figure 2, le dispositif comprend deux structures analogues à celles décrites précédemment, montées perpendiculairement l'une par rapport à l'autre. Ces deux structures sont parfaitement indépendantes l'une de l'autre. Toutefois, afin de s'assurer que la traction exercée s'effectue simultanément, on munit le dispositif d'un organe de verrouillage (29), agissant au niveau d'une patte (30) soudée sur chacun des arbres secondaires (22). Cet organe de verrouillage (29) est solidaire d'une poignée (31), qui lui est sensiblement perpendiculaire. Cette poignée (31) est articulé autour d'un axe (32) ménagé sur le bâti du dispositif, afin de permettre sa rotation et ainsi, l'escamotage de l'organe de verrouillage et donc la libération de la patte (30). Cette libération permet en outre la rotation des axes secondaires (22), et par voie de conséquence celle des axes primaires (21), compte tenu de leur dépendance par le biais du tirant (18).

Il est à noter que chaque dispositif comprend un organe de verrouillage (29) ainsi que les éléments avec lesquels ils coopèrent. De la sorte, et afin de permettre à un seul utilisateur de libérer les deux charges simultanément, et ainsi d'induire une traction immédiate au niveau de chacune des extrémités des deux bandes perpendiculaires, les deux poignées (31) des organes de verrouillage sont situées au voisinage l'une de l'autre.

Le dispositif ainsi décrit permet de réaliser simplement des mesures de propriétés mécaniques, notamment de fluage, et spécialement fluage mono-axial et bi-axial, et de ténacité à la traction pour des échantillons de matériau et ce sans requérir un matériel de coût élevé. De plus, la mise en oeuvre d'un tel matériel est aisé et ne demande pas de personnel qualifié.

## Revendications

1. Dispositif pour mesurer les propriétés mécaniques d'un échantillon sous forme de bande (1) fixée à ses extrémités (6, 7) entre deux mâchoires (8, 9), et reposant sur un socle (2) et comprenant :
- deux cylindres de renvoi parallèles (4, 5), disposés de part et d'autre du socle (2) et sur lesquels est renvoyée ladite bande (1), sur les deux extrémités libres (6, 7) de laquelle s'exerce une traction égale par l'intermédiaire des mâchoires (8, 9) ;
- deux biellettes pendulaires (12, 13) fixées respectivement d'une part aux mâchoires (8, 9), et d'autre part à deux équerres (14, 15) par l'intermédiaire de deux chapes pendulaires (16, 17), les deux équerres (14, 15) étant contenues dans le même plan et solidaires de deux axes parallèles, respectivement primaire et secondaire, et solidarisées entre elles au moyen d'un tirant dont les deux extrémités sont fixées aux équerres (14, 15) par l'intermédiaire de chapes, les deux équerres (14, 15) étant homothétiques l'une de l'autre par rapport au plan les contenant ;
- un bras de levier, solidaire de l'arbre primaire (21), dont la direction est toujours parallèle à la direction définie par la droite passant par respectivement l'axe de rotation de l'arbre primaire (21) et de l'arbre secondaire (22) et l'axe des pivotements des chapes solidarisant les biellettes (12, 13) aux équerres (14, 15) ;
- un porte-poids, fixé pendulairement à l'extrémité libre du bras de levier (25) et destiné à recevoir des masses de grandeur connue.

2. Dispositif selon la revendication 1, caractérisé en ce que les arbres primaire (21) et secondaire (22) sont montés sur des paliers munis de roulements à billes (23, 24).

3. Dispositif selon l'une des revendications 1 et 2, caractérisé en ce que la liaison des biellettes (12, 13) sur les mâchoires (8, 9) s'effectue au moyen de rotules.

4. Dispositif selon l'une des revendications 1 à 3, caractérisé en ce que les chapes sont fixées sur des roulements (23, 24).

5. Dispositif selon l'une des revendications 1 à 4, caractérisé en ce que le socle (2) est chauffant.

6. Dispositif selon l'une des revendications 1 à 5, caractérisé en ce que les mâchoires (8,9) sont auto-bloquantes et auto-serrantes.

7. Dispositif selon l'une des revendications 1 à 6, caractérisé en ce qu'il comprend un photomètre ou un système de traitement d'images, situé au dessus du socle (2) permettant de mesurer les propriétés mécaniques et notamment de fluage.

8. Dispositif pour mesurer les propriétés mécaniques d'un échantillon sous forme de bandes selon deux directions perpendiculaires, caractérisé en ce que la traction sur chacune des deux bandes perpendiculaires est effectuée au moyen de deux dispositifs similaires, indépendants l'un de l'autre, décrits dans les revendications 1 à 7.

9. Dispositif selon la revendication 8, caractérisé en ce que les bras de levier (25) sont verrouillés en position haute au moyen d'un organe de verrouillage (29) muni d'une poignée (31) articulée, le dit organe (29) coopérant avec une patte (30) solidaire des arbres secondaires (22).

## Patentansprüche

1. Vorrichtung zum Messen der mechanischen Eigenschaften einer Probe in Form eines Streifens (1), der an seinen Enden (6, 7) zwischen zwei Klemmen (8, 9) befestigt ist und auf einem Sockel (2) ruht, weist auf:
- zwei zueinander parallele Umlenkzylinder (4, 5), die beiderseits des Sockels (2) angeordnet sind und über die der Streifen (1) umgelegt ist, auf den über die Klemmen (8, 9) auf die beiden freien Enden (6, 7) eine Zugbeanspruchung gleicher Stärke ausgeübt ist,
- zwei bewegliche Stangen (12, 13), die jeweils auf der einen Seite an den Klemmen (8, 9) und an der anderen Seite an zwei Winkelstücken (14, 15) über zwei bewegliche Gabelstücke (16, 17) befestigt sind, wobei die beiden Winkelstücke (14, 15) in einer Ebene liegen und fest mit zwei, jeweils einer ersten und zweiten, parallelen Achse verbunden sind und untereinander über eine Zugstange verbunden sind, dessen zwei äußere Enden über Gabelstücke an den Winkelstücken (14, 15) angebracht sind, wobei die beiden Winkelstücke (14, 15) in der sie aufweisenden Ebene zueinander ähnlich sind,
- einen an der ersten Achse (21) fest angebrachten Hebelarm, dessen Richtung immer parallel zu der Richtung ist, die durch die jeweils durch die Drehachse der ersten Achse (21) und der zweiten Achse (22) und die Drehachse der die Stangen (12, 13) an den Winkelstücken 14, 15) befestigenden Gabelstücke verlaufende Gerade festgelegt ist und
- einen Gewichtsträger, der an dem freien Ende des Hebelarmes (25) befestigt ist und der zur Aufnahme von Massenstücken bekannter Größe dient.

2. Vorrichtung nach Anspruch 1, dadurch gekennzeichnet, daß die erste Achse (21) und zweite Achse (22) in Kugellager (23, 24) befestigt sind.

3. Vorrichtung nach einem der Ansprüche 1 und 2, dadurch gekennzeichnet, daß die Verbindung der Stangen (12, 13) mit den Klemmen (8, 9) mittels Gelenke ausgeführt ist.

4. Vorrichtung nach einem der Ansprüche 1 bis 3, dadurch gekennzeichnet, daß die Gabelstücke an Lager (23, 24) befestigt sind.

5. Vorrichtung nach einem der Ansprüche 1 bis 4, dadurch gekennzeichnet, daß der Sockel (2) heizbar ist.

6. Vorrichtung nach einem der Ansprüche 1 bis 5, dadurch gekennzeichnet, daß die Klemmen (8, 9) selbstverriegelnd und selbstverklemmend sind.

7. Vorrichtung nach einem der Ansprüche 1 bis 6, dadurch gekennzeichnet, daß sie ein über dem Sockel (2) angeordnetes Photometer oder ein Bildverarbeitungssystem aufweist, das das Messen der mechanischen Eigenschaften und insbesondere des Dehnverhaltens erlaubt.

8. Vorrichtung zum Messen der mechanischen Eigenschaften einer Probe in Form eines Streifens in zwei zueinander rechtwinkligen Richtungen, dadurch gekennzeichnet, daß die Zugbeanspruchung auf jeden der zueinander rechtwinkligen Streifen über zwei ähnliche, voneinander unabhängige, in den Ansprüchen 1 bis 7 beschriebenen Vorrichtungen ausgeübt ist.

9. Vorrichtung nach Anspruch 8, dadurch gekennzeichnet, daß die Hebelarme (25) in oberer Position mittels eine einen gelenkigen Griff (31) aufweisende Verriegelungseinheit (29) verriegelbar sind, wobei diese Einheit (29) mit einer an den zweiten Achsen (22) fest angebrachten Klaue (30) zusammenarbeiten.

## Claims

1. Apparatus for measuring the mechanical properties of a sample in web form (1) fixed at its ends (6, 7) between two jaws (8, 9) and resting on a baseplate (2), and comprising:
- two parallel return cylinders (4, 5), arranged on either side of the baseplate (2) and over which is returned the said web (1), an equal traction being exerted over both free ends (6, 7) of the said web by means of the jaws (8, 9);
- two hanging connecting rods (12, 13) respectively fixed, on the one hand, to the jaws (8, 9) and, on the other hand, to two brackets (14, 15) by means of two hanging clevises (16, 17), the two brackets (14, 15) being contained within the same plane and securely fastened to two parallel spindles, respectively a primary spindle and a secondary spindle, and securely fastened to one another by means of a tie rod whose two ends are fixed to the brackets (14, 15) by means of clevises, the two brackets (14, 15) being homothetic with one another with respect to the plane containing them;
- a lever arm, securely fastened to the primary shaft (21), whose direction is always parallel to the direction defined by the straight line passing respectively through the axis of rotation of the primary shaft (21) and of the secondary shaft (22) and the axis of pivoting of the clevises securely fastening the connecting rods (12, 13) to the brackets (14, 15);
- a weight carrier, fixed hanging from the free end of the lever arm (25) and intended to receive masses of known size.

2. Apparatus according to Claim 1, characterised in that the primary shaft (21) and secondary shaft (22) are mounted on bearings equipped with ball bearings (23, 24).

3. Apparatus according to either of Claims 1 and 2, characterised in that the connecting rods (12, 13) are linked to the jaws (8, 9) by means of ball joints.

4. Apparatus according to one of Claims 1 to 3, characterised in that the clevises are fixed onto roller-contact bearings (23, 24).

5. Apparatus according to one of Claims 1 to 4, characterised in that the baseplate (2) heats up.

6. Apparatus according to one of Claims 1 to 5, characterised in that the jaws (8, 9) are self-locking and self-clamping.

7. Apparatus according to one of Claims 1 to 6, characterised in that it comprises a light meter or an image processing system, situated above the baseplate (2) making it possible to measure mechanical properties, and particularly creep properties.

8. Apparatus for measuring the mechanical properties of a sample in web form, according to two perpendicular directions, characterised in that the traction on each of the two perpendicular webs is produced by means of two similar apparatuses, which are independent of one another and are described in Claims 1 to 7.

9. Apparatus according to Claim 8, characterised in that the lever arms (25) are locked in the top position by means of a locking member (29) equipped with an articulated handle (31), the said member (29) interacting with a tab (30) securely fastened to the secondary shafts (22).
